# EUROPEAN PATENT APPLICATION

(11) **EP 4 821 458 A1**
(43) Date of publication of application: **14.10.2026**
(21) Application number: 24910619.6
(22) Date of filing: 05.12.2024
(51) Int. Cl.: A61B 34/35

(54) **PARALLEL MOTION MECHANISM, SURGICAL INSTRUMENT, AND SURGICAL ROBOT**

(30) Priority: 29.12.2023 CN 202311871655
(71) Applicant: Cornerstone Technology (Shenzhen) Limited, Shenzhen Guandong (CN)
(72) Inventor: WANG, Zerui, Shenzhen, Guangdong 518066 (CN); HUANG, Qun, Shenzhen, Guangdong 518066 (CN)
(74) Representative: Balder IP Law, S.L.
(86) International application number: PCT/CN2024/137186
(87) International publication number: WO 2025/139687

(57) **Abstract**

A parallel motion mechanism, a surgical instrument, and a surgical robot are provided. The parallel motion mechanism comprises a proximal joint assembly, a distal joint assembly, at least one constraint cable, and a plurality of drive assemblies. A distal end of the proximal joint assembly is articulable relative to a proximal end of the proximal joint assembly along at least one plane, and each plane passes through a central axis of the parallel motion mechanism and defines a normal plane passing through the central axis. A distal end of the distal joint assembly is articulable relative to a proximal end of the distal joint assembly along the at least one plane. The at least one constraint cable is configured to maintain an orientation of the distal end of the distal joint assembly relative to the proximal end of the proximal joint assembly. The plurality of drive assemblies are configured to actuate the distal joint assembly, each of the plurality of drive assemblies comprises a distal flexible member, a proximal flexible member and a connector. The distal flexible member comprises a distal section located within the distal joint. The flexible member comprises a proximal section located within the proximal joint assembly. The connector couples the distal flexible member and the proximal flexible member, and a stiffness thereof is greater than the two flexible members. **In** a neutral state, the distal section and the proximal section are located on two sides of the normal plane, respectively.

## Description

### CROSS REFERENCE TO RELATED DISCLOSURES

The application claims priority to Chinese Patent Application No. 202311871655.1, entitled "PARALLEL MOTION MECHANISM, SURGICAL INSTRUMENT AND SURGICAL ROBOT," filed on December 29, 2023, which is incorporated by reference herein in its entirety.

### TECHNICAL FIELD

The present disclosure relates to the technical field of medical devices, and more specifically to a parallel motion mechanism, a surgical instrument including the same and a surgical robot including the same.

### BACKGROUND

In existing technology, a surgical instrument includes a parallel motion mechanism coupled between the end effector and the rear end of the surgical instrument, which enables the end effector of the surgical instrument to reach a desired operating position more conveniently. Meanwhile, the parallel motion mechanism changes the position of the end effector without changing its orientation. Compared with an articulated serial structure, the adoption of a parallel motion mechanism ensures a larger motion range of the end effector. Improving the actuation stiffness of the parallel motion mechanism is conductive to enhancing the motion precision of the mechanism.

### SUMMARY

A series of simplified concepts are introduced in the summary section, which will be further elaborated in the embodiments. The summary section of the present disclosure is not intended to limit the key features and essential technical features of the technical solution, let alone to determine the protection scope of the claimed technical solution.

In a first aspect, some embodiments of the present disclosure provide a parallel motion mechanism, which is applicable to a surgical instrument. The parallel motion mechanism includes a proximal joint assembly, a distal joint assembly, at least one constraint cable and a plurality of drive assemblies. A distal end of the proximal joint assembly is articulable relative to a proximal end of the proximal joint assembly along at least one plane. A respective plane of the at least one plane defines a normal plane passing through a central axis of the parallel motion mechanism and being perpendicular to the respective plane. The distal joint assembly is configured to couple to an end effector. A distal end of the distal joint assembly is articulable relative to a proximal end of the distal joint assembly along the at least one plane. The proximal end of the distal joint assembly is fixed to the distal end of the proximal joint assembly. The at least one constraint cable is configured to maintain an orientation of the distal end of the distal joint assembly relative to the proximal end of the proximal joint assembly. One end of the at least one constraint cable is fixed to the distal end of the distal joint assembly, and the other end of the at least one constraint cable is fixed to the proximal end of the proximal joint assembly. The plurality of drive assemblies are configured to actuate the distal joint assembly. Each of the plurality of drive assemblies includes a distal flexible member, a proximal flexible member and a connector. The distal flexible member is routed through the distal joint assembly to be fixed to the distal end of the distal joint assembly. The distal flexible member has a distal section disposed in the distal joint assembly. The proximal flexible member is routed through the proximal joint assembly to be configured to couple to a rear transmission device. The proximal flexible member has a proximal section disposed in the proximal joint assembly. The connector couples the distal flexible member and the proximal flexible member. A stiffness of the connector is greater than both a stiffness of the distal flexible member and a stiffness of the proximal flexible member.

In a second aspect, some embodiments of the present disclosure provide a surgical instrument. The surgical instrument includes the parallel motion mechanism according to any one of the above embodiments as described in the first aspect, an end effector coupled to the distal joint assembly of the parallel motion mechanism, and a rear transmission device coupled to an end of the proximal flexible member.

In a third aspect, some embodiments of the present disclosure provide a surgical robot. The surgical robot includes a robotic arm, and the surgical instrument according to any one of the above embodiments as described in the second aspect. The robotic arm is provided with an instrument drive device. The surgical instrument is removably coupled to the robotic arm. The instrument drive device is operatively coupled to the rear transmission device to control the proximal flexible member via the rear transmission device.

### BRIEF DESCRIPTION OF THE DRAWINGS

The accompanying drawings of the present disclosure are hereby incorporated as part of this disclosure to facilitate an understanding of the present disclosure. The drawings illustrate embodiments of the present disclosure and their descriptions, which serves to explain the principles of the present disclosure.

In the drawings:
FIG. 1 is a top schematic view of a surgical robot according to some embodiments of the present disclosure.
FIG. 2 is a side schematic view of the robotic arm system shown in FIG.1.
FIG. 3 is a schematic diagram of a surgical instrument according to some embodiments of the present disclosure.
FIG. 4 is a schematic diagram of partial members of the surgical instrument shown in FIG. 3, illustrating a distal joint assembly, an end joint assembly and an end effector.
FIG. 5 is a schematic diagram of the surgical instrument shown in FIG.3, with the shaft tube omitted.
FIG. 6 is a schematic diagram of partial members of the surgical instrument shown in FIG. 3, illustrating a proximal joint assembly, a distal joint assembly, an end joint assembly, an end effector and end drive cables.
FIG. 7 is a schematic diagram of partial members of a parallel motion mechanism according to some embodiments of the present disclosure, illustrating a proximal joint assembly, a distal joint assembly and at least one constraint cable.
FIG. 8 is a schematic diagram of partial members of a parallel motion mechanism according to some embodiments of the present disclosure, illustrating a proximal joint assembly, a distal joint assembly and a plurality of drive assemblies.
FIG. 9 is a schematic diagram of one of the plurality of drive assemblies shown in FIG. 8.
FIG. 10 is a schematic diagram of the connector shown in FIG. 9.
FIGS. 11 and 12 are schematic diagrams of variant examples of the connector shown in FIG. 10.
FIG. 13 is a schematic diagram of a parallel motion mechanism according to some embodiments of the present disclosure, with the shaft tube and the at least one constraint cable omitted, and the parallel motion mechanism is in a zero position state.
FIG. 14 is a side schematic view of the parallel motion mechanism shown in FIG. 3 along a direction parallel to the first plane, the first proximal joint portion and the first distal joint portion are deviated from a zero position angle.
FIG. 15 is a side schematic view of the parallel motion mechanism shown in FIG. 3 along a direction parallel to a second plane, the second proximal joint portion and the second distal joint portion are deviated from a zero position angle.
FIG. 16 is a schematic view taken along the direction L1 in FIG. 13.
FIG. 17 is a schematic view taken along the direction L2 in FIG. 13.
FIG. 18 is a schematic diagram of various arrangement forms of the plurality of connectors shown in FIG. 13 along the axial direction of the parallel motion mechanism.
FIG. 19 is a schematic diagram of the proximal joint assembly shown in FIG. 13.

### DETAILED DESCRIPTION OF THE EMBODIMENTS

In the following description, numerous specific details are set forth to provide a more thorough understanding of the present disclosure. However, it will be apparent to those skilled in the art that the present disclosure may be implemented without one or more of these details. In other cases, well-known technical features in the art are omitted to avoid obscuring the present disclosure.

For a thorough understanding of the present disclosure, detailed descriptions are provided in the following sections. It is appreciated that these embodiments are set forth to make the disclosure of the present application thorough and complete, and to fully convey the concepts of these exemplary embodiments to those skilled in the art. It will be apparent that the implementation of the embodiments of the present disclosure is not limited to the specific details familiar to those skilled in the art. Embodiments of the present disclosure are described in detail below. However, the present disclosure may also include other embodiments beyond these detailed descriptions.

Ordinal terms such as "first" and "second" cited in the present disclosure are merely identifiers and do not have any other meanings, such as a particular order, etc. Moreover, for example, the term "first component" does not by itself imply the existence of a "second component," and the term "second component" does not by itself imply the existence of a "first component." The terms such as "first," "second," and "third" do not indicate any order and may be construed as mere identifiers.

It should be noted that the terms "upper," "lower," "front," "rear," "left," "right," "inner," "outer," and similar terms used herein are for illustrative purposes only and are not intended to be limiting.

The terms "distal" and "proximal" cited in the present disclosure are directional terms commonly employed in the field of interventional medical devices, where "distal" refers to the end away from the operator during a surgical procedure, and "proximal" refers to the end close to the operator during a surgical procedure.

The terms "central," "parallel," "perpendicular," "aligned," and similar terms cited in the present disclosure need not be exact, but may include typical engineering tolerances.

The present disclosure provides a parallel motion mechanism, a surgical instrument including the parallel motion mechanism, and a surgical robot including the surgical instrument.

Embodiments of the present disclosure are described below with reference to the accompanying drawings, which illustrate representative embodiments and are not intended to limit the scope thereof.

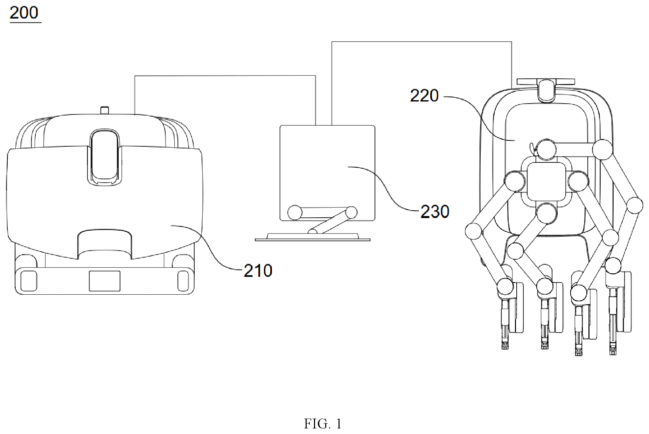

The surgical robot 200, according to some embodiments of the present disclosure, is a robot capable of performing surgery under remote manipulation. Referring to FIG. 1, the surgical robot 200 may include a control system 210 (also referred to as a surgeon's console 210), a robotic arm system 220 (also referred to as a patient-side robotic arm system 220), and an imaging system 230 (also referred to as an endoscopic system 230).

The control system 210 includes a display unit for presenting the surgical instrument environment, an operating control mechanism and an armrest. The display unit is provided with an observation window for viewing by the surgeon, the operating control mechanism is configured such that its movements correspond to those of the surgical instruments, and the armrest is configured to support the arms of the surgeon. In addition, the surgeon console 210 is equipped with additional control switches, conveniently positioned for hand or foot operation, enabling various functional commands to facilitate human-machine interaction.

The imaging system 230 includes a display screen, an endoscope controller, system electronics and an image processor.

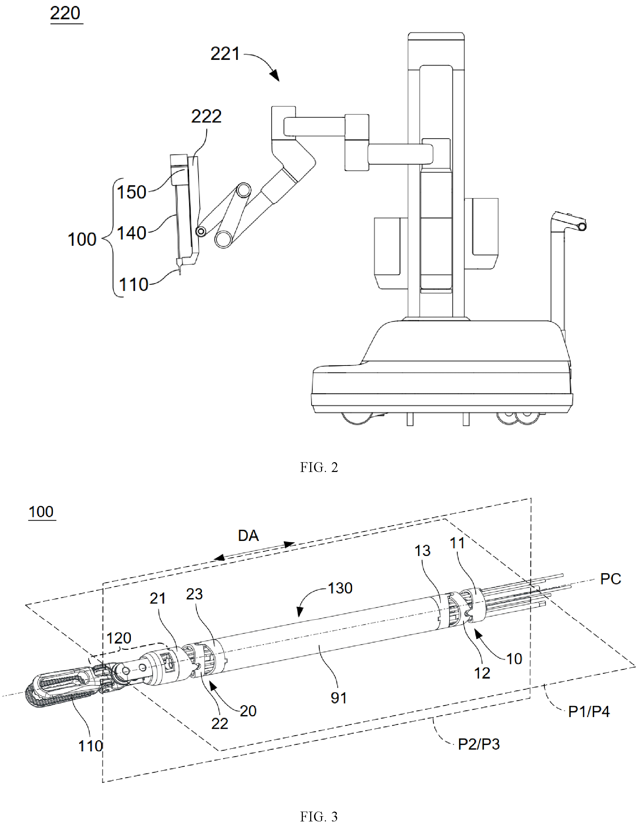

Referring to FIG. 2, the robotic arm system 220 may include at least one robotic arm 221 and a surgical instrument 100 mounted thereon. The robotic arm 221 is provided with a plurality of links, with two adjacent links being movable relative to each other with a specific degree of freedom, such that an end of the robotic arm 221 can achieve multi-degree-of-freedom movement (e.g., seven degrees of freedom, which may vary with different surgical instruments). An instrument holder 222 is disposed at the end of the robotic arm 221, and the surgical instrument 100 is removably mounted on the instrument holder 222. The surgical instrument 100 may be an instrument for performing surgical operations, such as an electrocautery device, forceps, a vessel sealer, and the like. The surgical instrument 100 may also be a camera for capturing images of the surgical area, such as an endoscope and the like. The surgical instrument 100 may also be other surgical instruments.

In some examples, the robotic arm 221 may be configured to move about a remote center of motion (RCM) maintained mechanically. For example, in a laparoscopic surgery, the RCM is aligned with a port through which the surgical instrument accesses an abdominal cavity of the patient. During the procedure of the surgery, the robotic arm 221 is manipulated to move the instrument holder 222 and thus to move the surgical instrument 100 to perform movements such as pitching, yawing, insertion and rolling. Throughout these movements, the longitudinal axis of the surgical instrument 100 is constrained to the RCM, thus preventing any non-surgical damage to the patient's abdominal incision caused by the surgical instrument 100.

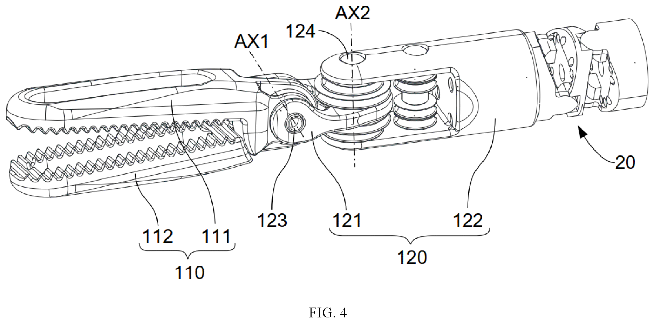

The surgical instrument 100 includes, from the proximal end to the distal end, a rear transmission device 150, a shaft 140 and an end effector 110. The rear transmission device 150 is engaged with an instrument drive device on the instrument holder 222. The rear transmission device 150 may be coupled to the end effector 110 via transmission members such as push-pull rods, cables, ropes and belts. The shaft 140 is coupled to and disposed between the rear transmission device 150 and the end effector 110 for maintenance of a certain distance of the rear transmission device 150 from the end effector 110 and for support of the end effector 110. The end effector 110 may be a tool such a hook, a spatula, a needle, forceps, scissors, for performing surgical operations such as tissue cutting, or an endoscopic lens for capturing image. For example, in an example of the present disclosure, as shown in FIG. 4, the end effector 110 is a pair of forceps, which includes a first jaw 111 and a second jaw 112 and is capable of performing opening and closing motions. When the surgical instrument 100 is electrosurgical, the surgical instrument 100 further includes an electrical cable, which is electrically connected to the end effector 110 for supplying electrical energy to the end effector 110.

Furthermore, to improve the movability of the end-effector 110, one or more joints may be disposed between the end-effector 110 and the shaft 140, such as a wrist, a parallel motion mechanism, an elbow and the like. The rear transmission device 150 may actuate the joint via transmission members such as push-pull rods, cables, ropes, and belts.

Referring to FIG. 3, the surgical instrument 100 according to an embodiment of the present disclosure includes an end joint assembly 120 and a parallel motion mechanism 130. The end effector 110 is coupled to the distal end of the wrist 120, and the proximal end of the wrist 120 is coupled to the distal end of the parallel motion mechanism 130. In an example of the present disclosure, the wrist 120 is disposed closer to the end effector 110 than other joints and is therefore also referred to as the end joint assembly 120 herein.

In an example of the present disclosure, the end joint assembly 120 includes a pitch joint and a yaw joint. Specifically, as shown in FIG. 4, the end joint assembly 120 may include a pitch joint base 122 and yaw joint base 121 that are coupled to each other. The yaw joint base 121 is configured to couple to the end effector 110. The pitch joint base 122 is configured to couple to the distal end of the parallel motion mechanism 130, so that the end joint assembly 120 and end effector 110 can move along with the distal end of the parallel motion mechanism 130. The proximal end of the end effector 110 is pivotably coupled to the distal end of the yaw joint base 121 about the yaw axis AX1, forming the yaw joint. For example, the proximal end of the end effector 110 is pivotably coupled to the distal end of the yaw joint base 121 via a first clevis pin 123, and the axis of the first clevis pin 123 is the yaw axis AX1. The proximal end of the yaw joint base 121 is pivotably coupled to the distal end of the pitch joint base 122 around the pitch axis AX2, forming the pitch joint. For example, the proximal end of the yaw joint base 121 is pivotably coupled to the distal end of the pitch joint base 122 via a second clevis pin 124, and the axis of the second clevis pin 124 is the pitch axis AX2. The axes AX1 and AX2 are non-parallel, for example, perpendicular to each other.

In an example of the present disclosure, the end effector 110 is configured as a pair of forceps. The end effector 110 includes a first jaw 111 and a second jaw 112 that are arranged oppositely. The proximal ends of the first jaw 111 and the second jaw 112 are pivotably coupled to the yaw joint base 121 via the first clevis pin 123, so that the first jaw 111 and the second jaw 112 are independently pivotable relative to the yaw joint base 121 about the first clevis pin 123.

For example, the yaw joint base 121 may be configured in a U-shape, with the proximal ends of the first jaw 111 and the second jaw 112 of the end effector 110 received within the U-shaped space of the yaw joint base 121 and pivotably coupled to the yaw joint base 121 via the first clevis pin 123. Rotation of the first jaw 111 and the second jaw 112 enables yaw movement of the end joint assembly 120 and opening and closing motions of the end effector 110. Similarly, the pitch joint base 122 may be configured in a U-shape, with the proximal end of the yaw joint base 121 received within the U-shaped space of the pitch joint base 122 and pivotably coupled thereto via the second clevis pin 124. Rotation of the yaw joint base 121 enables pitch movement of the end joint assembly 120.

It is appreciated that, in an example of the present disclosure, both the pitch joint and the yaw joint within the end joint assembly 120 are configured as pivot joints to reduce the length of the end joint assembly 120 and reduce the movement space of the end joint assembly 120. However, in other unillustrated embodiments, the pitch joint and the yaw joint may alternatively be configured as rolling joints, a combination of a pivot joint and a rolling joint, serpentine joints, or other arrangements.

It is appreciated that, in an example of the present disclosure, the end joint assembly 120 has pitch and yaw degrees of freedom. However, in other unillustrated embodiments, the end joint assembly 120 may include only one degree of freedom (e.g., either pitch or yaw) and alternatively, three or more degrees of freedom.

It is appreciated that, in other unillustrated embodiments, as variations of the present disclosure, the wrist 120 may be omitted between the end effector 110 and the parallel motion mechanism 130, and/or the elbow may be disposed between the parallel motion mechanism 130 and the rear transmission device 150.

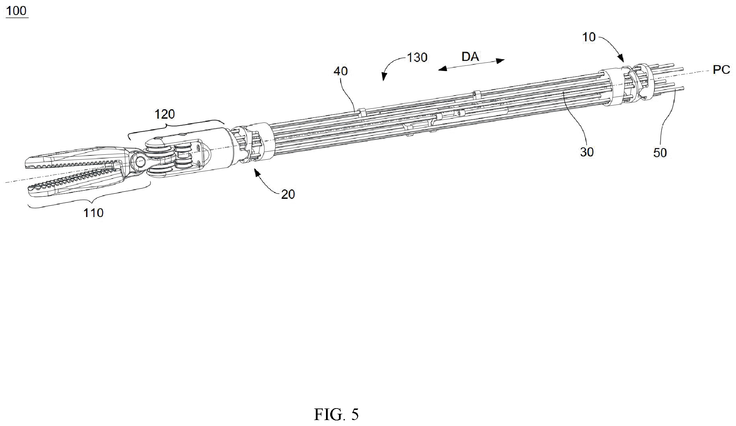

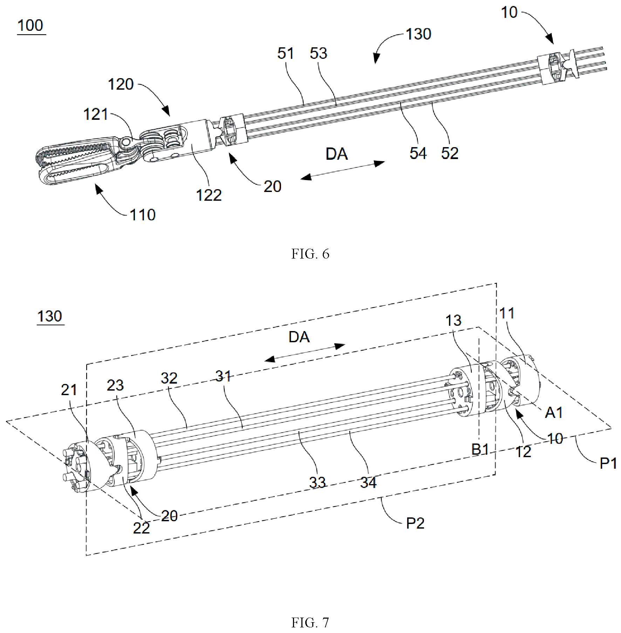

FIG. 5 illustrates transmission mechanisms through which the aforementioned rear transmission device actuates the parallel motion mechanism 130 and the end joint assembly 120. As shown in FIG. 5, the surgical instrument 100 further includes end drive cables 50. An instrument drive device (not shown) disposed on the instrument holder 222 actuates the end joint assembly 120 and/or the end effector 110 via the rear transmission device 150 and the end drive cables 50. The number of the end drive cables 50, is associated with the degrees of freedom of movement of the end joint assembly 120. Referring to FIG. 6, the end joint assembly 120 in this example has two degrees of freedom (i.e. pitch and yaw), so two pairs of end drive cables 50 may be provided. For example, one pair of end drive cables (51 and 52) control the pitch movement of the end joint assembly 120, while the other pair of end drive cables (53 and 54) control the yaw movement of the end joint assembly 120.

When the end effector 110 is a tool requiring opening and closing motion, such as a pair of forceps or scissors, the opening and closing motion of the end effector 110 may be controlled by controlling the pair of end drive cables (53 and 54) that control yaw movement. That is, the pair of end drive cables control the yaw movement and the opening/closing motion of the end effector 110. The specific control method, along with the specific structure and working principle of the rear transmission device 150, may refer to existing solutions, and will not be further elaborated herein. Alternatively, the opening and closing motion of the end effector 110 may be controlled by additional end drive cables other than the two pairs of end drive cables.

The end drive cables 50 may be configured as either metal cables (such as steel cable or tungsten cable) or drive ropes with high stiffness.

The parallel motion mechanism 130 includes a proximal joint assembly 10 and a distal joint assembly 20. The proximal joint assembly 10 is disposed at a proximal end of the parallel motion mechanism 130. The proximal joint assembly 10 is configured to couple to the shaft 140. The distal joint assembly 20 is disposed at a distal end of the parallel motion mechanism 130. The distal joint assembly 20 is configured to couple to the end effector 110. For example, the distal joint assembly 20 is coupled to the end effector 110 via the end joint assembly 120. The end effector 110 is coupled to the distal end of the end joint assembly 120, and the proximal end of the end joint assembly 120 is coupled to the distal end of the parallel motion mechanism 130. Thus, the end effector 110 and the distal joint assembly 20 are coupled via the end joint assembly 120. The proximal end of the distal joint assembly 20 is coupled to the distal end of the proximal joint assembly 10 and is relatively fixed with the distal end of the proximal joint assembly 10.

In one example, the proximal end of the distal joint assembly 20 is coupled to the distal end of the proximal joint assembly 10 via a shaft tube 91 and is relatively fixed with respect to the distal end of the proximal joint assembly 10. For example, the shaft tube 91 is constructed as a rigid member to maintain the shape of the shaft tube 91, which ensures that the relative position and orientation between the proximal end of the distal joint assembly 20 and the distal end of the proximal joint assembly 10 remain unchanged. The shaft tube 91 may be configured as an elongated cylindrical shape, which increases the operating radius of the parallel motion mechanism 130 while facilitating cable routing. When the parallel motion mechanism 130 is not deflected from its neutral position, i.e., in its zero position (also referred to as the neutral state), as shown in FIGS. 3 and 5 to 8, the proximal joint assembly 10 and the distal joint assembly 20 extend along the axial direction DA, so that the parallel motion mechanism 130 presents a straight structure. In this state, the central axis of the shaft tube 91 is coincident with the central axis PC of the parallel motion mechanism 130, and the direction where the central axis PC extends (hereafter, "extension direction") is the axial direction DA. The proximal end of the distal joint assembly 20 and the distal end of the proximal joint assembly 10 may be coupled to the shaft tube 91 by, for example, threading, adhesive bonding and snap-fitting. In other unillustrated examples, the shaft tube 91 may be omitted, such that the proximal end of the distal joint assembly 20 and the distal end of the proximal joint assembly 10 may be directly coupled to each other via threading, adhesive bonding, snap-fitting and similar means.

A distal end of the proximal joint assembly 10 is articulable relative to a proximal end of the proximal joint assembly 10 along at least one plane, where each plane passes through the central axis PC of the parallel motion mechanism 130 and is non-coincident with one another when there are multiple planes. A distal end of the distal joint assembly 20 is also articulable relative to a proximal end of the distal joint assembly 20 along the same at least one plane. The at least one plane may include a first plane P1 and/or a second plane P2 as shown in FIG. 3 and may include other planes that do not coincide with the first plane P1 and the second plane P2.

It should be noted that, as used herein, the description of a first member articulable relative to a second member along a plane is to be understood as meaning that the any part of the first member moves relative to the second member, lies within or is parallel to that plane.

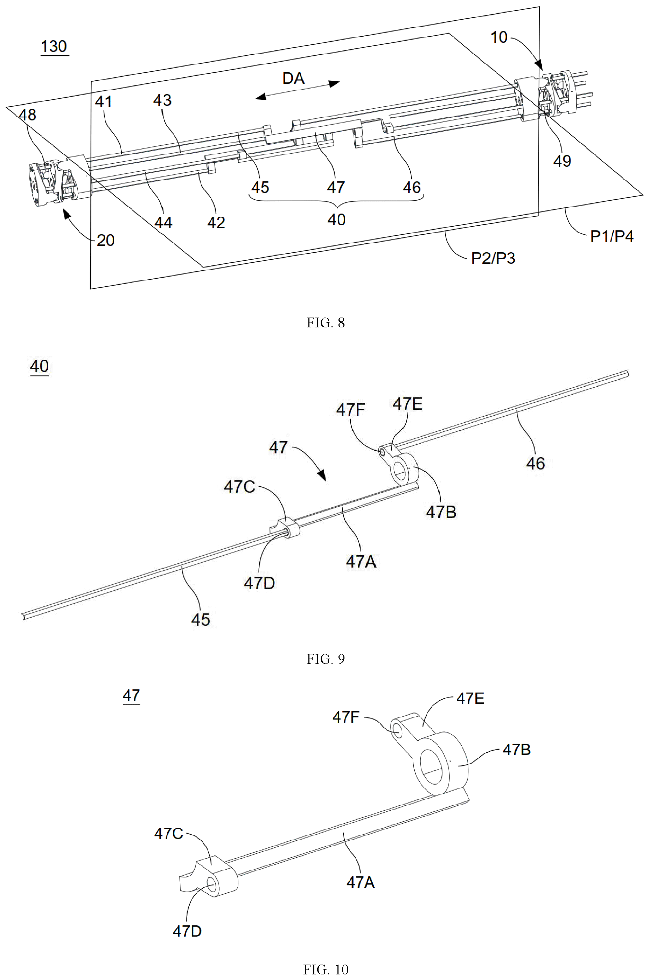

As shown in FIG. 5 and FIG. 7, the parallel motion mechanism 130 further includes at least one constraint cable 30. As shown in FIG. 5 and FIG. 8, the parallel motion mechanism 130 further includes a plurality of drive assemblies 40. The plurality of drive assemblies 40 are configured to actuate the distal joint assembly 20. The at least one constraint cable 30 is configured to maintain an orientation of the distal end of the distal joint assembly 20 relative to the proximal end of the proximal joint assembly 10. As the distal joint assembly 20 moves, the proximal joint assembly 10 is actuated by the at least one constraint cable 30 synchronously.

For example, one end of the at least one constraint cable 30 is fixed to the proximal end of the proximal joint assembly 10, and the other end of the at least one constraint cable 30 is fixed to the distal end of the distal joint assembly 20. To maintain the orientation of the distal end of the distal joint assembly 20 relative to the proximal end of the proximal joint assembly 10, the at least one constraint cable 30 may be configured such that the proximal joint assembly 10 and the distal joint assembly 20 are articulated at the same angle in opposite directions. The at least one constraint cable 30 keeps in tension within the parallel motion mechanism 130, thereby ensuring the synchronous movement of the proximal joint assembly 10 and the distal joint assembly 20. The at least one constraint cable 30 has a high stiffness, such that its length remains substantially unchanged when subjected to a tensile force that maintains its tensioned state. The at least one constraint cable 30 may be made of a rigid material, such as a steel cable, a tungsten cable and the like, or may be high-stiffness drive ropes.

The plurality of drive assemblies 40 are configured to actuate the distal end of the distal joint assembly 20 to swing relative to the proximal end of the distal joint assembly 20 along the at least one plane. Specifically, each drive assembly 40 has one end fixed to the distal end of the distal joint assembly 20, and the other end passed sequentially through the distal joint assembly 20, the shaft tube 91 and the proximal joint assembly 10 to be coupled to the rear transmission device 150 disposed at the proximal end of the surgical instrument 100. Thus, the instrument drive device disposed in the instrument holder 222 can actuate the distal joint assembly 20 via the rear transmission device 150 and the drive assemblies 40. When the instrument drive device outputs a driving force, the driving force is transmitted to the distal end of the distal joint assembly 20 via the rear transmission device 150 and the drive assemblies 40, so that the distal end of the distal joint assembly 20 is articulated relative to the proximal end of the distal joint assembly 20, and the distal end of the at least one constraint cable 30 is moved. Since the length of the constraint cable 30 remains unchanged, the proximal end of the at least one constraint cable 30 is moved correspondingly, which causes the proximal end of the constraint cable 30 to actuate the proximal end of the proximal joint assembly 10 and enables the proximal end of the proximal joint assembly 10 to swing relative to the distal end of the proximal joint assembly 10. At this time, the parallel motion mechanism 130 is in a shape where two ends bend in two opposite directions relative to the middle part, which is referred to as the deflection state of the parallel motion mechanism 130.

As shown in FIG. 8 and FIG. 9, each drive assembly 40 includes a distal flexible member 45, a proximal flexible member 46 and a connector 47.

The distal flexible member 45 is routed through the distal joint assembly 20 to be fixed to the distal end of the distal joint assembly 20. The distal flexible member 45 has a distal section 48 disposed in the distal joint assembly 20. The proximal flexible member 46 is routed through the proximal joint assembly 10 to be configured to couple to the rear transmission device 150. The proximal flexible member 46 has a proximal section 49 disposed in the proximal joint assembly 10. The distal flexible member 45 and the proximal flexible member 46 may be configured as drive cables, ropes or belts similar to the end drive cables 50 and/or the at least one constraint cable 30, such as steel cables, tungsten cables, steel belts and the like.

The connector 47 couples the distal flexible member 45 and the proximal flexible member 46. The stiffness of the connector 47 is greater than (furthermore, significantly greater than) both the stiffness of the distal flexible member 45 and the stiffness of the proximal flexible member 46. Accordingly, under identical tensile force, the deformation of the connector 47 is smaller than the deformation of the distal flexible member 45 and the proximal flexible member 46, or can be deemed negligible in comparison. For example, the connector 47 may be formed as a rigid member, such as a metal piece or a rigid plastic piece. The stiffness of the connector 47 may be enhanced by adjusting the bearing area and/or selecting different materials.

The end of the proximal flexible member 46 is coupled to the rear transmission device 150. The instrument drive device of the robotic arm 221 is engaged with the rear transmission device 150, so as to actuate the proximal flexible member 46 (e.g., pulling in or feeding out of the proximal flexible member 46) via the rear transmission device 150, and further actuate the drive assembly 40.

In the embodiments of the present disclosure, the distal end of the proximal joint assembly 10 is articulable relative to the proximal end of the proximal joint assembly 10 along at least one plane, and the distal end of the distal joint assembly 20 is also articulable relative to the proximal end of the distal joint assembly 20 along the same at least one plane. Each plane defines a normal plane passing through the central axis PC of the parallel motion mechanism and perpendicular to the associated plane. When the parallel motion mechanism 130 is in a neutral state, the distal section 48 and the proximal section 49 of each drive assembly 40 are disposed at two opposing sides of the normal plane, respectively. Thus, when the parallel motion mechanism 130is articulated, both the distal section 48 and the proximal section 49 increase in length, or both decrease in length. Compared with a case where the distal section 48 and the proximal section 49 are disposed on the same side of the normal plane, the variation in length of each drive assembly 40 within the parallel motion mechanism 130 is enlarged. This increases the transmission ratio and thus the displacement of each drive assembly 40, thereby reducing the driving force required for the rear transmission device 150. This reduces the tensile force borne by each drive assembly 40, which helps to mitigate tensile deformation of each drive assembly 40, thereby reducing transmission errors and improving drive precision.

In one example, when the parallel motion mechanism 130 is in a neutral state, the proximal section 49 and the distal section 48 of each drive assembly 40 extend parallel to the central axis PC, and a distance from the proximal section 49 to the normal plane is equal to a distance from the distal section 48 to the normal plane. Thus, the proximal section 49 and the distal section 48 increase or decrease equally in length, which enables the transmission ratio to be increased by an integer multiple. This design facilitates simplification of the structural design and drive control of the mechanism.

In one example, when the parallel motion mechanism 130 is in a neutral state, the proximal section 49 and the distal section 48 of each drive assembly 40 extend parallel to the central axis PC. When the parallel motion mechanism 130 is projected onto a plane perpendicular to an extension direction of the central axis PC (in the axial direction DA), the projections of the proximal section 49 and the distal section 48 of each drive assembly 40 are symmetrically arranged about the central axis PC. This configuration is advantageous for the parallel motion mechanisms 130 with multiple degrees of freedom the number of which determines the number of the plurality of drive assemblies 40, as it facilitates the design for the routing paths of the flexible transmission members of multiple drive assemblies 40, and avoids crossing and motion interference among the flexible transmission members.

Since the distal section 48 and the proximal section 49 of each drive assembly 40 are disposed at two opposing sides of the normal plane, respectively, the distal flexible member 45 and the proximal flexible member 46 tend to extend curvedly or helically in the parallel motion mechanism 130. This extension form lowers the overall stiffness of each drive assembly 40 and is unfavorable to driving precision improvement. To guarantee the overall stiffness of each drive assembly 40, each drive assembly 40 is configured such that the distal flexible member 45 and the proximal flexible member 46 extend straightly between the proximal end of the distal joint assembly 20 and the distal end of the proximal joint assembly 10. Specifically, the distal flexible member 45 extends along a first straight line between the proximal end of the distal joint assembly 20 and the distal end of the proximal joint assembly 10, and the proximal flexible member 46 extends along a second straight line between the proximal end of the distal joint assembly 20 and the distal end of the proximal joint assembly 10.

In one example, the first straight line and the second straight line may lie in the same plane, such that the driving force from the rear transmission device 150 is transmitted in this plane, which can improve the transmission efficiency of the driving force. Optionally, the plane where the first straight line and the second straight line lie in may pass through the central axis PC, such that the first straight line, the second straight line and the central axis PC are coplanar, which can further enhance transmission efficiency.

In one embodiment, the first straight line and the second straight line may both be parallel to the central axis PC of the parallel motion mechanism 130. On the one hand, this configuration reduces the lengths of the distal flexible member 45 and the proximal flexible member 46, thereby minimizing the tensile deformation of each drive assembly 40. On the other hand, this configuration is advantageous with the case where there are multiple drive assemblies 40, as it facilitates the design for the routing paths of the flexible transmission members of multiple drive assemblies 40, and avoids crossing and motion interference among the flexible transmission members.

To ensure that the distal flexible member 45 and the proximal flexible member 46 both extend straightly between the proximal end of the distal joint assembly 20 and the distal end of the proximal joint assembly 10, the connection positions of the connector 47 with the two flexible members are circumferentially spaced around the central axis PC. Specifically, as shown in FIG. 9 and FIG.10, the connector 47 includes a first connection position 47D coupled to the distal flexible member 45 and a second connection position 47F coupled to the proximal flexible member 46, where the first connection position 47D and the second connection position 47F are circumferentially spaced about the central axis PC.

In one example, when the parallel motion mechanism 130 is in a neutral state, and when the parallel motion mechanism 130 is projected onto a plane perpendicular to the extension direction DA of the central axis PC, a line connecting the projections of the distal flexible member 45 and the proximal section 49 intersects both the first connection position 47D and the second connection position 47F, so that the first straight line and the second straight line are coplanar. Optionally, the line may further intersect the central axis PC, thereby making the first straight line, the second straight line and the central axis PC coplanar.

In one example, when the parallel motion mechanism 130 is in a neutral state, and when the parallel motion mechanism 130 is projected onto a plane perpendicular to the extension direction DA of the central axis PC, the projection of the first connection position 47D is coincident with the projection of the distal flexible member 45, and the projection of the second connection position 47F is coincident with the projection of the proximal section 49, such that both the first straight line and the second straight line are parallel to the central axis PC.

In one example, the first connection position 47D and the second connection position 47F are spaced apart in the extension direction DA of the central axis PC. This increases the length proportion of the connector 47 in each drive assembly 40, thereby contributing to an increase in the stiffness of each drive assembly 40.

Three exemplary structures of the connector 47 are described below with reference to FIGS. 10 to 12.

As shown in FIG. 10 to FIG. 12, the connector 47 includes a first connection portion 47C configured to couple to the distal flexible member 45. Specifically, the distal flexible member 45 is coupled to a first connection position 47D of the first connection portion 47C. Similarly, the connector 47 further includes a second connection portion 47E configured to couple to the proximal flexible member 46. Specifically, the proximal flexible member 46 is coupled to a second connection position 47F of the second connection portion 47E. The connector 47 further includes a connecting rod 47A. The first connection portion 47C and the second connection portion 47E are connected via the connecting rod 47A. For example, the connecting rod 47A may extend along the central axis PC and have two ends, namely a distal end and a proximal end. The first connection portion 47C is disposed at the distal end of the connecting rod 47A, and the second connection portion 47E is disposed at the proximal end of the connecting rod 47A.

The parallel motion mechanism 130 further includes a central tube 92. The central tube 92 extends along the extension direction DA of the central axis PC and is configured to couple between the proximal joint assembly 10 and the distal joint assembly 20 to guide other cables, such as cables for supplying power to the end effector 110. For example, the central tube 92 is also constructed as a rigid member, to maintain the shape of the central tube 92. The axis of the central tube 92 is coincident with the central axis PC of the parallel motion mechanism 130. A plurality of connectors 47 of the plurality of drive assemblies 40 of the parallel motion mechanism 130 are arranged circumferentially around the central tube 92. In other words, the plurality of drive assemblies 40 are circumferentially spaced and disposed in the annular space between the shaft tube 91 and the central tube 92 of the parallel motion mechanism 130. Optionally, the plurality of drive assemblies 40 are circumferentially spaced at equal intervals along the parallel motion mechanism 130.

The connector 47 may further include a guide portion 47B, which is configured to couple to the central tube 92 to counteract a couple moment exerted on the connector 47 by the distal flexible member 45 and the proximal flexible member 46. The guide portion 47B is movable relative to the central tube 92 along the extension direction DA of the central axis PC. For example, the guide portion 47B may be configured as a guide ring, which surrounds an outer circumference of the central tube 92. The guide ring 47B may be disposed at an end or a middle part of the connecting rod 47A. Accordingly, the first connection portion 47C may protrude radially from the outer peripheral surface of either the guide ring 47B or the connecting rod 47A, and the second connection portion 47E may protrude radially from the outer peripheral surface of either the guide ring 47B or the connecting rod 47A.

As shown in FIG. 10, the connector 47 includes a guide ring 47B disposed at the proximal end of the connecting rod 47A. The second connection portion 47E is coupled to the outer peripheral surface of the guide ring 47B and protrudes radially outward from the outer peripheral surface of the guide ring 47B, while the first connection portion 47C is coupled to the distal end of the connecting rod 47A and protrudes radially outward from the outer peripheral surface of the distal end. Alternatively, the guide ring 47B may be disposed at the distal end of the connecting rod 47A, with the first connection portion 47C protruding radially outward from the outer peripheral surface of the guide ring 47B and the second connection portion 47E protruding radially outward from the outer peripheral surface of the proximal end of the connecting rod 47A.

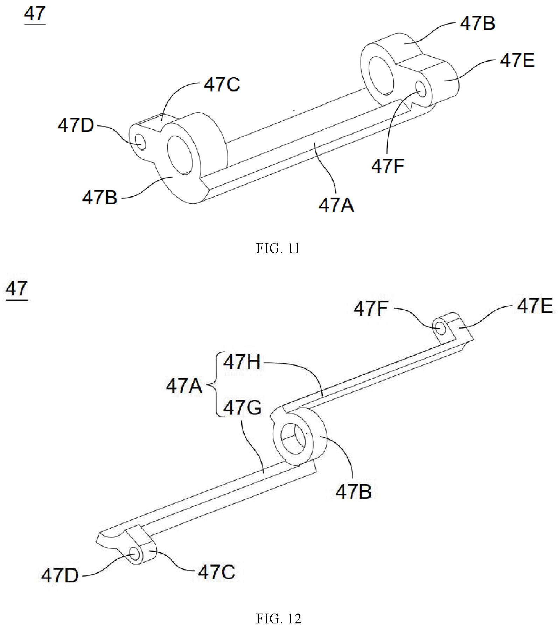

As shown in FIG. 11, the connector 47 includes two guide rings 47B, which are disposed at the proximal end and distal end of the coupling rod 47A, respectively. It is appreciated that the two guide rings 47B are coaxially arranged, and the central axes of the two guide rings 47B are coincident with the central axis PC. The first connection portion 47C is coupled to the outer peripheral surface of the distal guide ring 47B and protrudes radially outward from the outer peripheral surface of the distal guide ring 47B, and the second connection portion 47E is coupled to the outer peripheral surface of the proximal guide ring 47B and protrudes radially outward from the outer peripheral surface of the distal guide ring 47B.

As shown in FIG. 12, the connector 47 includes a guide ring 47B disposed at the middle part of the connecting rod 47A. The first connection portion 47C and the second connection portion 47E are respectively disposed at the two ends of the connecting rod 47A, and protrude radially outward from the outer peripheral surface of the connecting rod 47A. The connecting rod 47A includes a first rod segment 47G and a second rod segment 47H located on two opposing sides of the guide ring 47B along the axial direction DA. Optionally, the first rod segment 47G and the second rod segment 47H are circumferentially spaced about the guide ring 47B to facilitate the arrangement of the first connection portion 47C and the second connection portion 47E.

In the present disclosure, the outer peripheral surface of the connecting rod 47A refers to the side surface connecting the two end surfaces of the connecting rod 47A. The cross-sectional shape of the connecting rod 47A may be a portion of an annulus, or another shape adapted to the guide ring 47B or the central tube 92. For example, as shown in FIGS. 11 and 12, the side of the connecting rod 47A facing the guide ring 47B is configured as a concave surface conforming to the arc of the outer surface of the guide ring 47B. Thus, when the guide ring 47B is sleeved on the central tube 92, a gap is formed between the connecting rod 47A and the central tube 92, which prevents interference with the movement of the connector 47 relative to the central tube 92.

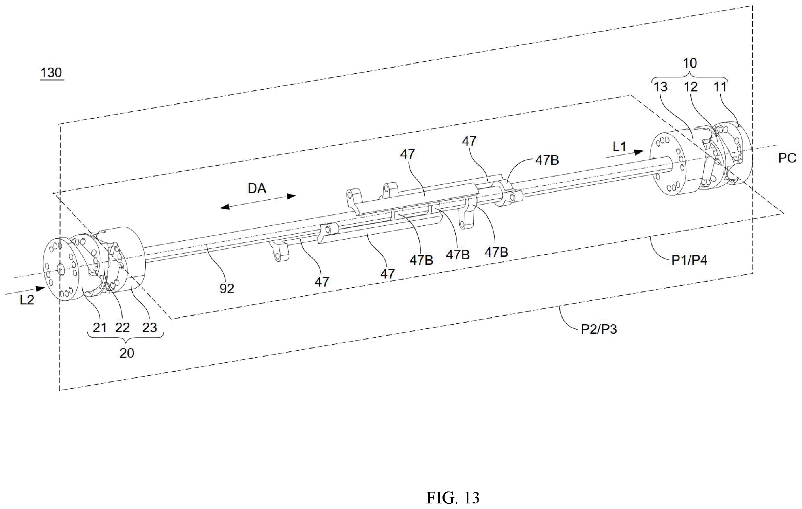

The plurality of connectors 47 may be arranged in a non-overlapping manner along the extension direction DA of the central axis PC, which helps to avoid interference between the plurality of connectors 47. Alternatively, at least two connectors 47 may be arranged in an overlapping manner along an extension direction of the central axis, which helps to shorten the length of the parallel motion mechanism 130. The plurality of connectors 47 configured as shown in FIGS. 10 and 12 are suitable for use in cases where the plurality of connectors 47 are arranged in an overlapping manner. For example, as shown in FIG. 13, four connectors 47 configured as illustrated in FIG. 10 are arranged in an overlapping manner. The plurality of connectors 47 configured as illustrated in FIG. 11 are suitable for cases where the plurality of connectors 47 are arranged in a non-overlapping manner, or where two connectors 47 are arranged in an overlapping manner in groups.

Embodiments of the present disclosure are described in detail below by taking the parallel motion mechanism 130 with two movable degrees of freedom as an example.

In a first degree of freedom of the parallel motion mechanism 130, the distal end of the proximal joint assembly 10 is articulable relative to the proximal end of the proximal joint assembly 10 along the first plane P1. Similarly, the distal end of the distal joint assembly 20 is articulable relative to the proximal end of the distal joint assembly 20 along the first plane P1, where the normal plane of the first plane P1 is a first normal plane P3. The plurality of drive assemblies 40 include a first drive assembly 41 and a second drive assembly 42. It is appreciated that the first drive assembly 41 and the second drive assembly 42 each include, as discussed above, the distal flexible member, the proximal flexible member, and the connector 47, which will not be repeated hereafter.

When the parallel motion mechanism 130 is in a neutral state, the proximal section and the distal section of the first drive assembly 41 are disposed at two opposing sides of the first normal plane P3, respectively; and the proximal section and the distal section of the second drive assembly 42 are disposed at two opposing sides of the first normal plane P3, respectively. The proximal section of the first drive assembly 41 and the proximal section of the second drive assembly 42 are disposed at two opposing sides of the first normal plane P3, respectively. Thus, the distal section of the first drive assembly 41 and the distal section of the second drive assembly 42 are also disposed at two opposing sides of the first plane P3, respectively.

In a second degree of freedom of the parallel motion mechanism 130, the distal end of the proximal joint assembly 10 is further articulable relative to the proximal end of the proximal joint assembly 10 along a second plane P2. Similarly, the distal end of the distal joint assembly 20 is articulable relative to the proximal end of the distal joint assembly 20 along the second plane P2, where the normal plane of the second plane P2 is a second normal plane P4. In an embodiment of the present disclosure, the first plane P1 and the second plane P2 intersect perpendicularly at the central axis PC of the parallel motion mechanism 130. The plurality of drive assemblies 40 include a third drive assembly 43 and a fourth drive assembly 44. It is appreciated that the third drive assembly 43 and the fourth drive assembly 44 each include, as discussed above, the distal flexible member, the proximal flexible member, and the connector 47, which will not be repeated hereafter.

When the parallel motion mechanism 130 is in a neutral state, the proximal section and the distal section of the third drive assembly 43 are disposed at two opposing sides of the second normal plane P4, respectively; and the proximal section and the distal section of the fourth drive assembly 44 are disposed at two opposing sides of the second normal plane P4, respectively. The proximal section of the third drive assembly 43 and the proximal section of the fourth drive assembly 44 are disposed at two opposing sides of the second normal plane P4, respectively. Thus, the distal section of the third drive assembly 43 and the distal section of the fourth drive assembly 44 are also disposed at two opposing sides of the second normal plane P4, respectively.

It is appreciated that, in an embodiment of the present disclosure where the first plane P1 is perpendicular to the second plane P2, the first normal plane P3 is coincident with the second plane P2, and the second normal plane P4 is coincident with the first plane P1.

In an embodiment of the present disclosure, the proximal joint assembly 10 includes a first proximal joint portion 11, a second proximal joint portion 12 and a third proximal joint portion 13 coupled in sequence. The first proximal joint portion 11 is disposed at the proximal end of the parallel motion mechanism 130. The second proximal joint portion 12 is coupled to the distal end of the first proximal joint portion 11 and is articulable relative to the first proximal joint portion 11 along the second plane P2. In other words, the movement trajectory of any point on the second proximal joint portion 12 relative to the first proximal joint portion 11 lies on or is parallel to the second plane P2. The third proximal joint portion 13 is coupled to the distal end of the second proximal joint portion 12 and is articulable relative to the second proximal joint portion 12 along the first plane P1. In other words, the movement trajectory of any point on the third proximal joint portion 13 relative to the second proximal joint portion 12 lies on or is parallel to the first plane P1.

The distal joint assembly 20 includes a first distal joint portion 21, a second distal joint portion 22 and a third distal joint portion 23 coupled in sequence. The third distal joint portion 23 is fixed to the third proximal joint portion 13, so as to maintain the relative position and orientation therebetween unchanged. The second distal joint portion 22 is coupled to the distal end of the third distal joint portion 23 and is articulable relative to the third distal joint portion 23 along the first plane P1. In other words, the movement trajectory of any point on the second distal joint portion 22 relative to the third distal joint portion 23 lies on or is parallel to the first plane P1. The first distal joint portion 21 is coupled to the distal end of the second distal joint portion 22 and is articulable relative to the second distal joint portion 22 along the second plane P2. In other words, the movement trajectory of any point on the first distal joint portion 21 relative to the second distal joint portion 22 lies on or is parallel to the second plane P2.

The third distal joint portion 23 and the third proximal joint portion 13 are coupled via the shaft tube 91. The shape of the shaft tube 91 remains unchanged, such that the relative position and the orientation between the third distal joint portion 23 and the third proximal joint portion 13 remain unchanged.

Optionally, in an embodiment of the present disclosure, as shown in FIG. 7, the parallel motion mechanism 130 is provided with two pairs of constraint cables 30, including a first constraint cable 31, a second constraint cable 32, a third constraint cable 33 and a fourth constraint cable 34. When the parallel motion mechanism 130 is in a neutral state, the two pairs of constraint cables 30 are symmetrically arranged with respect to both the first plane P1 and the second plane P2, and may extend parallel to the central axis PC. As each constraint cable has a fixed length, this arrangement causes each constraint cable 30 having its length change in the distal joint assembly 20 opposite to its length change in the proximal joint assembly 10. This achieves parallel motion of the parallel motion mechanism 130, and decouples motions in two degrees of freedom.

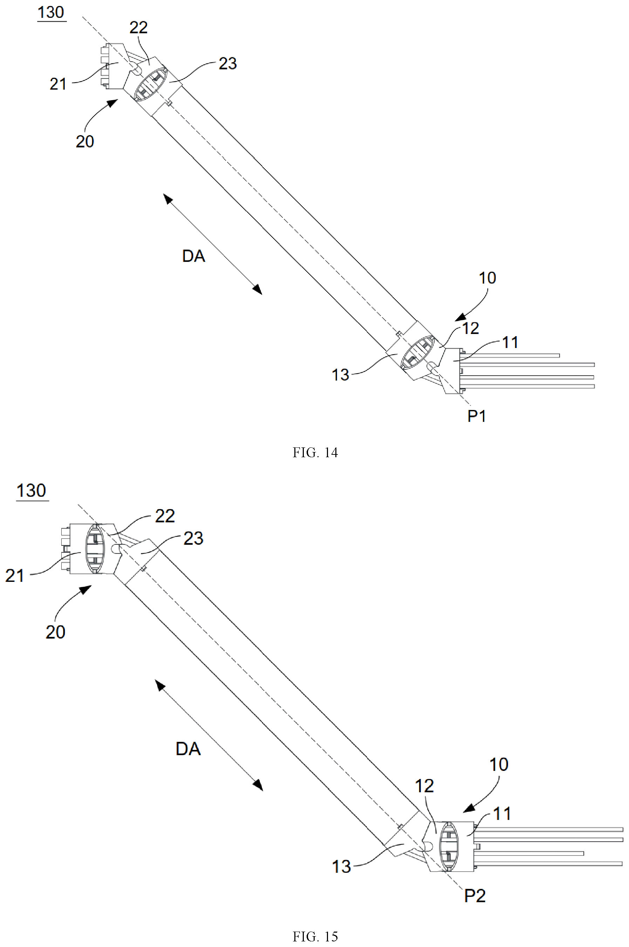

Specifically, when the parallel motion mechanism 130 moves in the first degree of freedom, the first distal joint portion 21 is articulated by a first angle relative to the second distal joint portion 22, with one side moving away from the second distal joint portion 22 and the other side moving closer to the second distal joint portion 22 (as shown in FIG. 14). For example, each of the first constraint cable 31 and the second constraint cable 32 increases in length between the first distal joint portion 21 and the second distal joint portion 22 by a first length, and decreases in length between the second proximal joint portion 12 and the first proximal joint portion 11 by the first length. Meanwhile, each of the third constraint cable 33 and the fourth constraint cable 34 decreases in length between the first distal joint portion 21 and the second distal joint portion 22 by the first length, and increases in length between the second proximal joint portion 12 and the first proximal joint portion 11 by the first length. As a result, the second proximal joint portion 12 is also articulated by the first angle relative to the first proximal joint portion 11 in the rotational direction opposite to that of the first distal joint portion 21 relative to the second distal joint portion 22.

When the parallel motion mechanism 130 moves in the second degree of freedom, the second distal joint portion 22 is articulated by a second angle relative to the third distal joint portion 23, with one side moving away from the third distal joint portion 23 and the other side moving closer to the third distal joint portion 23 (as shown in FIG. 15). For example, each of the first constraint cable 31 and the fourth constraint cable 34 increases in length between the second distal joint portion 22 and the third distal joint portion 23 by a second length, and decreases in length between the third proximal joint portion 13 and the second proximal joint portion 12 by the second length. Meanwhile, each of the second constraint cable 32 and the third constraint cable 33 decreases in length between the second distal joint portion 22 and the third distal joint portion 23 by the second length, and increases in length between the third proximal joint portion 13 and the second proximal joint portion 12 by the second length. As a result, the third proximal joint portion 13 is also articulated by the second angle relative to the second proximal joint portion 12 in the rotational direction opposite to that of the second distal joint portion 22 relative to the third distal joint portion 23.

Therefore, during the articulation of the parallel motion mechanism 130, the first proximal joint portion 11 and the first distal joint portion 21 are always kept parallel to each other (or in a consistent orientation therebetween) without changing the orientation of the end effector 110, thereby enabling translational movement of the end effector 110. In the present embodiment, the end effector 110 may translationally moves in two degrees of freedom.

In one example, the distance from the proximal section of the first drive assembly 41 to the first normal plane P3 is equal to the distance from the proximal section of the second drive assembly 42 to the first normal plane P3, and the distance from the distal section of the first drive assembly 41 to the first normal plane P3 is equal to the distance from the distal section of the second drive assembly 42 to the first normal plane P3. Thus, in a case where the parallel motion mechanism 130 is articulable in the first degree of freedom, the first drive assembly 41 increases in length in the parallel motion mechanism 130 by a length the same as that of the second drive assembly 42 decreasing in length in the parallel motion mechanism 130, or the first drive assembly 41 decreases in length in the parallel motion mechanism 130 by a length the same as that of the second drive assembly 42 increasing in length in the parallel motion mechanism 130. In other words, the total length of the first drive assembly 41 and the second drive assembly 42 in the parallel motion mechanism 130 is conserved, which further facilitates the control of the mechanism.

Optionally, the proximal section of the first drive assembly 41 and the proximal section of the second drive assembly 42 are symmetrically arranged about the central axis PC, and the distal section of the first drive assembly 41 and the distal section of the second drive assembly 42 are also symmetrically arranged about the central axis PC. Thus, in a case where the parallel motion mechanism 130 is articulable in both the first and second degrees of freedom, the first drive assembly 41 increases in length in the parallel motion mechanism 130 by a length the same as that of the second drive assembly 42 decreasing in length in the parallel motion mechanism 130, or the first drive assembly 41 decreases in length in the parallel motion mechanism 130 by a length the same as that of the second drive assembly 42 increasing in length in the parallel motion mechanism 130. In other words, the total length of the first drive assembly 41 and the second drive assembly 42 in the parallel motion mechanism 130 is conserved, which further facilitates the control of the mechanism.

Further optionally, the proximal section of the first drive assembly 41, the proximal section of the second drive assembly 42, the distal section of the first drive assembly 41 and the distal section of the second drive assembly 42 are all disposed in the first plane P1. Thus, in a case where the parallel motion mechanism 130 is articulable in both the first and second degrees of freedom, since the first plane P1 and the second plane P2 are perpendicular to each other, the length variation of the first drive assembly 41 and the length variation of the second drive assembly 42 are decoupled from the movement of the parallel motion mechanism 130 in the second degree of freedom.

Accordingly, the rear transmission device 150 may include a first capstan. The proximal flexible member of the first drive assembly 41 and the proximal flexible member of the second drive assembly 42 are two cables wound in opposite directions on the first capstan. When the parallel motion mechanism 130 is in a neutral state, the first drive assembly 41 and the second drive assembly 42 are substantially equal in length. As the first capstan rotates, the feeding-out (pulling-in) length of the proximal flexible member of the first drive assembly 41 is equal to the pulling-in (feeding-out) length of the proximal flexible member of the second drive assembly 42.

In one example, the distance from the proximal section of the third drive assembly 43 to the second normal plane P4 is equal to the distance from the proximal section of the fourth drive assembly 44 to the second normal plane P4, and the distance from the distal section of the third drive assembly 43 to the second normal plane P4 is equal to the distance from the distal section of the fourth drive assembly 44 to the second normal plane P4. Thus, in a case where the parallel motion mechanism 130 is articulable in the second degree of freedom, the third drive assembly 43 increases in length in the parallel motion mechanism 130 by a length the same as that of the fourth drive assembly 44 decreasing in length in the parallel motion mechanism 130, or the third drive assembly 43 decreases in length in the parallel motion mechanism 130 by a length the same as that of the fourth drive assembly 44 increasing in length in the parallel motion mechanism 130. In other words, the total length of the third drive assembly 43 and the fourth drive assembly 44 in the parallel motion mechanism 130 is conserved, which facilitates the control of the mechanism.

Optionally, the proximal section of the third drive assembly 43 and the proximal section of the fourth drive assembly 44 are symmetrically arranged about the central axis PC, and the distal section of the third drive assembly 43 and the distal section of the fourth drive assembly 44 are symmetrically arranged about the central axis PC. Thus, in a case where the parallel motion mechanism 130 is articulable in both the first and second degrees of freedom, the third drive assembly 43 increases in length in the parallel motion mechanism 130 by a length the same as that of the fourth drive assembly 44 decreasing in length in the parallel motion mechanism 130, or the third drive assembly 43 decreases in length in the parallel motion mechanism 130 by a length the same as that of the fourth drive assembly 44 increasing in length in the parallel motion mechanism 130. In other words, the total length of the third drive assembly 43 and the fourth drive assembly 44 in the parallel motion mechanism 130 is conserved, which further facilitates the control of the mechanism.

Further optionally, the proximal section of the third drive assembly 43, the proximal section of the fourth drive assembly 44, the distal section of the third drive assembly 43 and the distal section of the fourth drive assembly 44 are all disposed in the second plane P2. Thus, in a case where the parallel motion mechanism 130 is articulable in both the first and second degrees of freedom, since the first plane P1 and the second plane P2 are perpendicular to each other, the length variation of the third drive assembly 43 and the length variation of the fourth drive assembly 44 are decoupled from the movement of the parallel motion mechanism 130 in the first degree of freedom.

Accordingly, the rear transmission device 150 may include a second capstan. The proximal flexible member of the third drive assembly 43 and the proximal flexible member of the fourth drive assembly 44 are two cables wound in opposite directions on the second winch. When the parallel motion mechanism 130 is in a neutral state, the third drive assembly 43 and the fourth drive assembly 44 are substantially equal in length. As the second capstan rotates, the feeding-out (pulling-in) length of the proximal flexible member of the third drive assembly 43 is equal to the pulling-in (feeding-out) length of the proximal flexible member of the fourth drive assembly 44.

The end drive cables 50 are configured to drive the end joint assembly 120 and the end effector 110 to move. One end of each end drive cable 50 is coupled to the rear transmission device 150, and the other end of each end drive cable 50 extends through the shaft 140 and the parallel motion mechanism 130 to be coupled to the end joint assembly 120 or the end effector 110. To prevent the movement of the parallel motion mechanism 130 from affecting the control of the end joint assembly 120 and the end effector 110 by the end drive cables 50, it is necessary to decouple the end drive cables 50 from the parallel motion mechanism 130. For this purpose, the end drive cables 50 may be arranged such that, when the parallel motion mechanism 130 is in a neutral state, each pair of the end drive cables 50 extends parallel to the central axis PC and is arranged symmetrically about the central axis PC. Such an arrangement ensures that when the parallel motion mechanism 130 moves, the end drive cable 50 increases in length at the proximal joint assembly 10 by a length the same as that of the end drive cable 50 decreasing in length at the distal joint assembly 20, or the end drive cable 50 decreases in length at the proximal joint assembly 10 by a length the same as that of the end drive cable 50 increasing in length at the distal joint assembly 20. In other words, the length of each end drive cable 50 in the parallel motion mechanism 130 is conserved. That is, the movement of the parallel motion mechanism 130 does not cause a change in the length of each end drive cable 50, thereby achieving decoupling between each end drive cable 50 and the parallel motion mechanism 130.

In an example of the present disclosure, the end joint assembly 120 has two degrees of freedom including pitch and yaw. The movement of the end joint assembly 120 is controlled by two pairs of end drive cables 50. The two pairs of end drive cables are respectively a first end drive cable 51 and a second end drive cable 52, a third end drive cable 53 and a fourth end drive cable 54. When the parallel motion mechanism 130 is in a neutral state, the four end drive cables 50 extend parallel to the central axis PC in the parallel motion mechanism 130. The first end drive cable 51 and the second end drive cable 52 are symmetrically arranged about the central axis PC, and the third end drive cable 53 and the fourth end drive cable 54 are symmetrically arranged about the central axis PC.

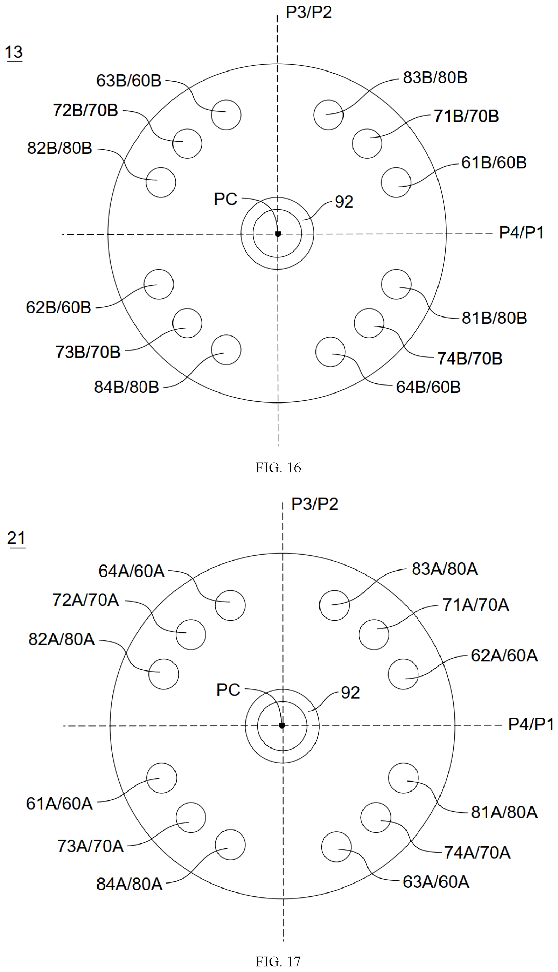

As described above, in an embodiment of the present disclosure, the surgical instrument 100 includes four constraint cables 30, four drive assemblies 40 and four end drive cables 50. All twelve members are routed sequentially through the first proximal joint portion 11, the second proximal joint portion 12, the third proximal joint portion 13, the third distal joint portion 23, the second distal joint portion 22 and the first distal joint portion 21. Thus, as shown in FIGS. 16 and 17, each of the first proximal joint portion 11, second proximal joint portion 12 and third proximal joint portion 13 is provided with four proximal constraint cable through-holes 70B for routing of the constraint cables 30, four proximal parallel motion drive cable through-holes 60B for routing of the drive assemblies 40, and four proximal end drive cable through-holes 80B for routing of the end drive cables 50. Each of the third distal joint portion 23, second distal joint portion 22, and first distal joint portion 21 is correspondingly provided with four distal constraint cable through-holes 70A for routing of the constraint cables 30, four distal parallel motion drive cable through-holes 60A for routing of the drive assemblies 40, and four distal end drive cable through-holes 80A for routing of the end drive cables 50.

Optionally, in the neutral state, the segments of each drive assembly 40 at the proximal joint assembly 10 and at the distal joint assembly 20 extend parallel to the central axis PC. Furthermore, as mentioned above, in the neutral state, each constraint cable 30 and each end drive cable 50 also extend parallel to the central axis PC. Thus, the set of through-holes (including three through-holes in the present embodiment) respectively arranged at the first proximal joint portion 11, the second proximal joint portion 12 and the third proximal joint portion 13 for routing each cable are aligned in a direction parallel to the central axis PC. Likewise, the three through-holes at the third distal joint portion 23, the second distal joint portion 22 and the first distal joint portion 21 for routing each cable are also aligned in a direction parallel to the central axis PC. In addition, the two through-holes at the third proximal joint portion 13 and the first distal joint portion 21 for each of the constraint cables 30 or the end drive cables 50 are aligned in a direction parallel to the central axis PC.

The arrangement of the twelve members is described below, with reference to the twelve through-holes of the third proximal joint portion 13 (see FIG. 16) and the twelve through-holes of the first distal joint portion 21 (see FIG. 17). FIGS. 16 and 17 respectively illustrate the third proximal joint portion 13 and the first distal joint portion 21 when the parallel motion mechanism 130 is in the neutral state, with the first plane P1, the second plane P2, and the central axis PC perpendicular to the plane of the paper.

For the four end drive cables 50, the proximal joint assembly 10 is provided with four sets of proximal end drive cable through-holes 80B. Taking the third proximal joint portion 13 as an example and with reference to FIG. 16, these are the first proximal end drive cable through-hole 81B, the second proximal end drive cable through-hole 82B, the third proximal end drive cable through-hole 83B, and the fourth proximal end drive cable through-hole 84B, respectively. The distal joint assembly 20 is provided with four sets of distal end drive cable through-holes 80A. Taking the first distal joint portion 21 as an example, with reference to FIG. 17, these are the first distal end drive cable through-hole 81A, the second distal end drive cable through-hole 82A, the third distal end drive cable through-hole 83A, and the fourth distal end drive cable through-hole 84A, respectively. The first proximal end drive cable through-hole 81B and the first distal end drive cable through-hole 81A are configured for passage of the first end drive cable 51. The second proximal drive cable through-hole 82B and the second distal end drive cable through-hole 82A are configured for passage of the second end drive cable 52. The third proximal drive cable through-hole 83B and the third distal end drive cable through-hole 83A are configured for passage of the third end drive cable 53. The fourth proximal drive cable through-hole 84B and the fourth end drive cable through-hole 84A are configured for passage of the fourth end drive cable 54.

As can be seen from FIG. 16, the through-holes 81B, 82B, 83B, and 84B are equidistant from the central axis PC. The through-holes 81B and 82B are arranged symmetrically about the central axis PC, while the through-holes 83B and 84B are arranged symmetrically about the central axis PC. For example, the through-holes 81B, 82B, 83B, and 84B are arranged rotationally symmetric about the central axis PC. As shown in FIG. 17, the arrangement of through-holes 81A, 82A, 83A, and 84A in the distal joint assembly 20 is analogous to that in the proximal joint assembly 10 as depicted in FIG. 16, and thus will not be repeated herein.

Furthermore, it can be seen with reference to FIGS. 16 and 17 that the through-holes 81B, 82B, 83B, and 84B of the proximal joint assembly 10 are aligned, respectively, with the through-holes 81A, 82A, 83A, and 84A of the distal joint assembly 20 along the direction parallel to the central axis PC. Accordingly, in the neutral state, the four end drive cables 51, 52, 53, and 54 extend parallel to the central axis PC in the parallel motion mechanism 130.

For the four constraint cables 30, the proximal joint assembly 10 is provided with four sets of proximal constraint cable through-holes 70B. Taking the third proximal joint portion 13 as an example and with reference to FIG. 16, these are respectively the first proximal constraint cable through-hole 71B, the second proximal constraint cable through-hole 72B, the third proximal constraint cable through-hole 73B, and the fourth proximal constraint cable through-hole 74B. The distal joint assembly 20 is provided with four sets of distal constraint cable through-holes 70A. Taking the first distal joint portion 21 as an example, with reference to FIG. 17, these are respectively the first distal constraint cable through-hole 71A, the second distal constraint cable through-hole 72A, the third distal constraint cable through-hole 73A, and the fourth distal constraint cable through-hole 74A. The first proximal constraint cable through-hole 71B and the first distal constraint cable through-hole 71A are configured for passage of the first constraint cable 31. The second proximal constraint cable through-hole 72B and the second distal constraint cable through-hole 72A are configured for passage of the second constraint cable 32. The third proximal constraint cable through-hole 73B and the third distal constraint cable through-hole 73A are configured for passage of the third constraint cable 33. The fourth proximal constraint cable through-hole 74B and the fourth distal constraint cable through-hole 74A are configured for passage of the fourth constraint cable 34.

As can be seen from FIG. 16, the through-holes 71B, 72B, 73B, and 74B are arranged rotationally symmetric about the central axis PC. The through-holes 71B, 72B, 73B, and 74B are symmetrically arranged with respect to both the first plane P1 and the second plane P2, thereby ensuring that the four constraint cables 30 are symmetrically arranged with respect to both the first plane P1 and the second plane P2 in the proximal joint assembly 10. Furthermore, each of the through-holes 71B, 72B, 73B, and 74B is equidistant from the first plane P1 and the second plane P2. In other words, the line connecting the center of each through-hole 71B, 72B, 73B, and 74B to the central axis PC bisects the angle formed between the first plane P1 and the second plane P2. As can be seen from FIG. 17, the arrangement of the through-holes 71A, 72A, 73A, and 74A in the distal joint assembly 20 is similar to that in the proximal joint assembly 10 shown in FIG. 16 and will not be described in detail herein.

Furthermore, as can be seen from FIGS. 16 and 17, the through-holes 71B, 72B, 73B, and 74B of the proximal joint assembly 10 are aligned, respectively, with the through-holes 71A, 72A, 73A, and 74A of the distal joint assembly 20 in the direction parallel to the central axis PC. Thus, in the neutral state, the four constraint cables 31, 32, 33, and 34 extend parallel to the central axis PC in the parallel motion mechanism 130.

For the four drive assemblies 40, the proximal joint assembly 10 is provided with four sets of proximal parallel motion drive cable through-holes 60B. Taking the third proximal joint portion 13 as an example with reference to FIG. 16, such holes include a first proximal parallel motion drive cable through-hole 61B, a second proximal parallel motion drive cable through-hole 62B, a third proximal parallel motion drive cable through-hole 63B and a fourth proximal parallel motion drive cable through-hole 64B. The distal joint assembly 20 is provided with four sets of distal parallel motion drive cable through-holes 60A. Taking the first distal joint portion 21 as an example with reference to FIG. 17, such holes include a first distal parallel motion drive cable through-hole 61A, a second distal parallel motion drive cable through-hole 62A, a third distal parallel motion drive cable through-hole 63A and a fourth distal parallel motion drive cable through-hole 64A. The first proximal parallel motion drive cable through-hole 61B is configured for passage of the proximal flexible member of the first drive assembly 41, and the first distal parallel motion drive cable through-hole 61A is configured for passage of the distal flexible member of the first drive assembly 41. The second proximal parallel motion drive cable through-hole 62B is configured for passage of the proximal flexible member of the second drive assembly 42, and the second distal parallel motion drive cable through-hole 62A is configured for passage of the distal flexible member of the second drive assembly 42. The third proximal parallel motion drive cable through-hole 63B is configured for passage of the proximal flexible member of the third drive assembly 43, and the third distal parallel motion drive cable through-hole 63A is configured for passage of the distal flexible member of the third drive assembly 43. The fourth proximal parallel motion drive cable through-hole 64B is configured for passage of the proximal flexible member of the fourth drive assembly 44, and the fourth distal parallel motion drive cable through-hole 64A is configured for passage of the distal flexible member of the fourth drive assembly 44.

Unlike the through-holes 70B, 70A, 80B, and 80A, the proximal parallel motion drive cable through-holes 60B are not aligned, respectively, with the distal parallel motion drive cable through-holes 60A in the direction parallel to the central axis PC.

Specifically, when the parallel motion mechanism 130 is projected onto a plane perpendicular to the axial direction DA, the projections of the first proximal parallel motion drive cable through-hole 61B and the first distal parallel motion drive cable through-hole 61A are symmetrically arranged about the central axis PC, such that the projections of the proximal section of the first drive assembly 41 and the distal section of the first drive assembly 41 are symmetrically arranged about the central axis PC. The second proximal parallel motion drive cable through-hole 62B and the second distal parallel motion drive cable through-hole 62A are symmetrically arranged about the central axis PC, such that the projections of the proximal section of the second drive assembly 42 and the distal section of the second drive assembly 42 are symmetrically arranged about the central axis PC. The first proximal parallel motion drive cable through-hole 61B and the second proximal parallel motion drive cable through-hole 62B are symmetrically arranged about the central axis PC, such that the projections of the proximal section of the first drive assembly 41 and the proximal section of the second drive assembly 42 are symmetrically arranged about the central axis PC. The first distal parallel motion drive cable through-hole 61A and the second distal parallel motion drive cable through-hole 62A are symmetrically arranged about the central axis PC, such that the projections of the distal section of the first drive assembly 41 and the distal section of the second drive assembly 42 are symmetrically arranged about the central axis PC.

Where the first plane P1 is perpendicular to the second plane P2, the first proximal parallel motion drive cable through-hole 61B is aligned with the second distal parallel motion drive cable through-hole 62A, and the first distal parallel motion drive cable through-hole 61A is aligned with the second proximal parallel motion drive cable through-hole 62B.

Similarly, when the parallel motion mechanism 130 is projected onto a plane perpendicular to the axial direction DA, the projections of the third proximal parallel motion drive cable through-hole 63B and the third distal parallel motion drive cable through-hole 63A are symmetrically arranged about the central axis PC, such that the projections of the proximal section of the third drive assembly 43 and the distal section of the third drive assembly 43 are symmetrically arranged about the central axis PC. The fourth proximal parallel motion drive cable through-hole 64B and the fourth distal parallel motion drive cable through-hole 64A are symmetrically arranged about the central axis PC, such that the projections of the proximal section of the fourth drive assembly 44 and the distal section of the fourth drive assembly 44 are symmetrically arranged about the central axis PC. The third proximal parallel motion drive cable through-hole 63B and the fourth proximal parallel motion drive cable through-hole 64B are symmetrically arranged about the central axis PC, such that the projections of the proximal section of the third drive assembly 43 and the proximal section of the fourth drive assembly 44 are symmetrically arranged about the central axis PC. The third distal parallel motion drive cable through-hole 63A and the fourth distal parallel motion drive cable through-hole 64A are symmetrically arranged about the central axis PC, such that the projections of the distal section of the third drive assembly 43 and the distal section of the fourth drive assembly 44 are symmetrically arranged about the central axis PC.

Where the first plane P1 is perpendicular to the second plane P2, the third proximal parallel motion drive cable through-hole 63B is aligned with the fourth distal parallel motion drive cable through-hole 64A, and the third distal parallel motion drive cable through-hole 63A is aligned with the fourth proximal parallel motion drive cable through-hole 64B.

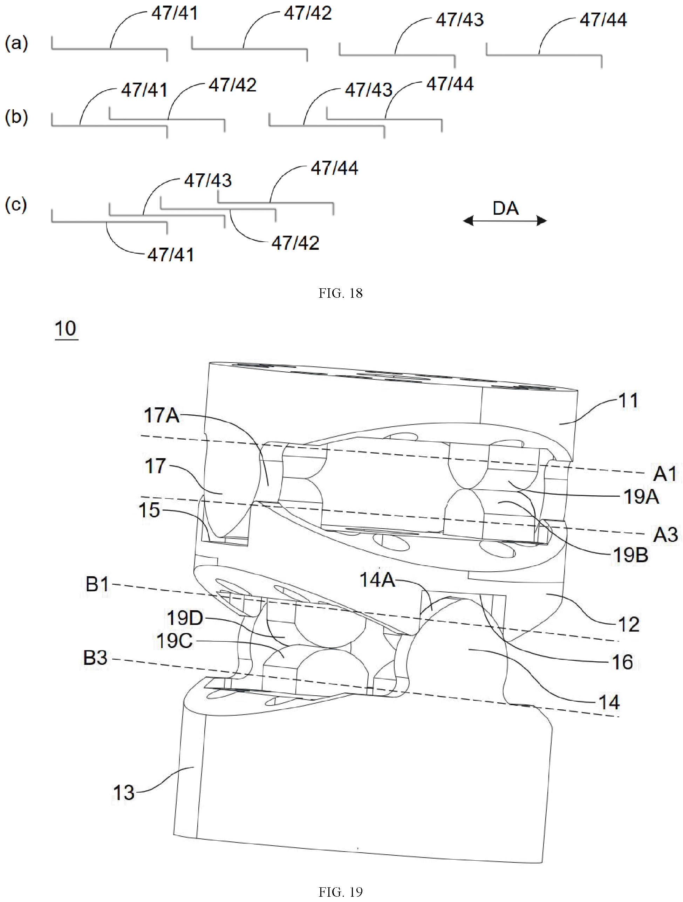

The present embodiment is provided with four drive assemblies 40 and correspondingly four connectors 47. The arrangement of the four connectors 47 is shown in FIG. 18. FIG. 18(a) shows that the four connectors 47 are arranged in a non-overlapping manner along the extension direction DA of the central axis PC. FIG. 18(b) shows the connector 47 of the first drive assembly 41 and the connector 47 of the second drive assembly 42 are arranged in an overlapping manner along the extension direction DA of the central axis PC, and the connector 47 of the third drive assembly 43 and the connector 47 of the fourth drive assembly 44 are arranged in an overlapping manner along the extension direction DA of the central axis PC. FIG. 18(c) illustrates all four connectors 47 arranged to overlapped with one another along the extension direction DA of the central axis PC. It is appreciated that the sequence of the four connectors 47 is not limited to the illustrated arrangement and can be adjusted as needed.

In some embodiments of the present disclosure, the proximal joint assembly 10 and the distal joint assembly 20 may be configured as rolling joints. With reference to FIG. 19, by taking the proximal joint assembly 10 as an example, the motion mechanism of the rolling joints and the schematic structure for implementing the same are briefly described.

As shown in FIG. 19, the second proximal joint portion 12 is provided with first engagement recesses 15 on two opposing sides of the second proximal joint portion 12, respectively, and the first proximal joint portion 11 is provided with first teeth 17 on two opposing sides of the first proximal joint portion 11, respectively. The first teeth 17 are engaged with the first engagement recesses 15. When relative rotation occurs between the first proximal joint portion 11 and the second proximal joint portion 12, the first tooth 17 rotates within the first engagement recess 15. Concurrently, edges defining an opening of the first engagement recess 15 are kept in contact with the edges 17A of the first tooth 17.

Additionally, the first proximal joint portion 11 is provided with a first rolling surface 19A, and the second proximal joint portion 12 is provided with a second rolling surface 19B. When relative rotation occurs between the first proximal joint portion 11 and the second proximal joint portion 12, the first rolling surface 19A and the second rolling surface 19B are kept in contact with and roll against each other. Serving as a load-bearing mechanism, the first rolling surface 19A and the second rolling surface 19B withstand the interaction force between the first proximal joint portion 11 and the second proximal joint portion 12 induced by tensile load from the drive assemblies. Specifically, each of the first rolling surface 19A and the second rolling surface 19B is configured as a circular arc surface. The axis of the circular arc of the first rolling surface 19A is the first proximal axis A1, while the axis of the circular arc of the second rolling surface 19B is the third proximal axis A3. The third proximal axis A3 extends parallel to the first proximal axis A1 and maintains a constant distance from the first proximal axis A1. The first proximal axis A1 and the third proximal axis A3 are perpendicular to the second plane P2.

Benefiting from the profile shape of the first tooth edge 17A and the fit relation between the first tooth 17 and the first engagement recess 15, the first rolling surface 19A and the second rolling surface 19B perform relative rigid pure rolling free of relative sliding as the first tooth 17 rotates within the first engagement recess 15. When the first rolling surface 19A undergoes rigid pure rolling on the second rolling surface 19B in the first rotational direction, the first rolling surface 19A (or any part of the first proximal joint portion 11) revolves about the third proximal axis A3 by a first angle in the first rotational direction, and is simultaneously rotating about the first proximal axis A1 by the same first angle in the first rotational direction. As a result, relative rotation (articulation) between the first proximal joint portion 11 and the second proximal joint portion 12 is realized.

Similar to the rotation between the first proximal joint portion 11 and the second proximal joint portion 12, relative rotation between the second proximal joint portion 12 and the third proximal joint portion 13 also realized via rigid pure rolling of matching components. For example, the third proximal joint portion 13 is provided with a second tooth 14, and the second proximal joint portion 12 is provided with a second engagement recess 16 configured to engage with the second tooth 14. Meanwhile, the second proximal joint portion 12 is provided with an arc-shaped fourth rolling surface 19D, and the third proximal joint portion 13 is provided with a corresponding arc-shaped third rolling surface 19C that performs rigid pure rolling against the fourth rolling surface 19D. The axis of the circular arc of the fourth rolling surface 19D is the second proximal axis B1, while the axis of the circular arc of the third rolling surface 19C is the fourth proximal axis B3. The third rolling surface 19C and the fourth rolling surface 19D serve as a load-bearing mechanism to withstand interaction forces between the third proximal joint portion 13 and the second proximal joint portion 12 induced by tensile loads from the drive assemblies. The second proximal axis B1 and the fourth proximal axis B3 extend parallel to each other and maintain a constant distance therebetween. The second proximal axis B1 and the fourth proximal axis B3 are perpendicular to the first plane P1. Benefiting from the contour geometry of the second tooth 14A and the fitting cooperation between the second tooth 14 and the second engagement recess 16, the third rolling surface 19C and the fourth rolling surface 19D realize relative rigid pure rolling free of relative sliding as the second tooth 14 rotates within the second engagement recess 16.

The relative rotational (articulable) motion mechanism between the third proximal joint portion 13 and the second proximal joint portion 12 is similar to the aforementioned mechanism between the first proximal joint portion 11 and the second proximal joint portion 12, and which will not repeated herein.

For example, the first engagement recess 15 and the second engagement recess 16 are angularly offset by 90 degrees, so the first proximal axis A1 extends perpendicular to the second proximal axis B1.

Similarly, the distal joint assembly 20 may also adopt the identical motion mechanism and corresponding structural configuration as the proximal joint assembly 10, which will not be repeated herein.

In addition to the structure shown in FIG. 19, the rolling joint may be implemented in other forms. For example, the rolling surfaces arranged on two adjacent joint portions may be omitted, and a connecting rod may be arranged between the two adjacent joint portions, with two ends of the connecting rod being rotationally coupled to the two adjacent joint portions respectively, so that the connecting rod functions as the load-bearing mechanism. Alternatively, the teeth and the engagement recesses formed on the two adjacent joint portions may be substituted with intermeshing gear pairs.

Although the embodiments of the present disclosure specifically describe a parallel motion mechanism 130 as having two degrees of freedom, it is appreciated that in other embodiments, the parallel motion mechanism 130 may alternatively have one degree of freedom or more than two degrees of freedom.

In interpreting the scope of the present disclosure, the term "comprising" and its derivatives as used herein are intended to be open-ended terms, which specify the presence of the described features, elements, components, groups, assemblies, and/or steps, but do not exclude the presence of other unrecited features, elements, components, groups, assemblies, and/or steps. This concept also applies to terms with similar meanings, such as "including," "having," and their derivatives.

The term "attached" or "affixed" as used herein includes: a configuration in which an element is directly secured to another element by fixing it directly thereto; a configuration in which an element is indirectly secured to another element by fixing it to an intermediate member, which is in turn secured to another element; and a configuration in which one element is integral with another, i.e., one element is essentially part of another. This definition also applies to words with similar meanings, such as "connect," "join," "couple," "mount," "bond," "fix," and their derivatives. Finally, degree terms such as "substantially," "approximately," and "roughly" used herein denote deviations that modify the terms without significantly altering the final result.

Unless otherwise defined, the technical and scientific terms used herein have the same meanings as those commonly understood by a person skilled in the art in the technical field of the present disclosure. The terminology used herein is for the sole purpose of describing specific embodiments and is not intended to limit the scope of the present disclosure. Features described in one embodiment may be applied separately or in combination with other features in another embodiment, unless such a feature is inapplicable to that other embodiment or is otherwise specified.

The present disclosure has been described with reference to the above embodiments, but it is appreciated that the embodiments are provided merely for the purpose of illustration and description and are not intended to limit the present disclosure to the scope of the described embodiments. Furthermore, persons skilled in the art will appreciate that the present disclosure is not limited to the embodiments described above, and that numerous variations and modifications may be made in accordance with the teachings of the present disclosure, all of which fall within the scope of protection claimed by the present disclosure.

## Claims

1. A parallel motion mechanism, applicable to a surgical instrument, the parallel motion mechanism comprising:
a proximal joint assembly, wherein a distal end of the proximal joint assembly is articulable relative to a proximal end of the proximal joint assembly along at least one plane, and a respective plane of the at least one plane defines a normal plane passing through a central axis of the parallel motion mechanism and being perpendicular to the respective plane;
a distal joint assembly configured to couple to an end effector, wherein a distal end of the distal joint assembly is articulable relative to a proximal end of the distal joint assembly along the at least one plane, and the proximal end of the distal joint assembly is fixed to the distal end of the proximal joint assembly;
at least one constraint cable configured to maintain an orientation of the distal end of the distal joint assembly relative to the proximal end of the proximal joint assembly, wherein one end of the at least one constraint cable is fixed to the distal end of the distal joint assembly, and the other end of the at least one constraint cable is fixed to the proximal end of the proximal joint assembly; and
a plurality of drive assemblies configured to actuate the distal joint assembly, each of the plurality of drive assemblies including:
a distal flexible member routed through the distal joint assembly to be fixed to the distal end of the distal joint assembly, the distal flexible member having a distal section disposed in the distal joint assembly;
a proximal flexible member routed through the proximal joint assembly to be configured to couple to a rear transmission device, the proximal flexible member having a proximal section disposed in the proximal joint assembly; and
a connector coupling the distal flexible member and the proximal flexible member, a stiffness of the at least one connector being greater than both a stiffness of the distal flexible member and a stiffness of the proximal flexible member.

2. The parallel motion mechanism according to claim 1, wherein, when the parallel motion mechanism is in a neutral state, the proximal section and the distal section are disposed at two opposing sides of the normal plane, respectively.

3. The parallel motion mechanism according to claim 2, wherein, when the parallel motion mechanism is in the neutral state, the proximal section and the distal section extend parallel to the central axis, and a distance from the proximal section to the normal plane is equal to a distance from the distal section to the normal plane.

4. The parallel motion mechanism according to claim 2, wherein, when the parallel motion mechanism is in the neutral state, in a projection of the parallel motion mechanism along an extension direction of the central axis, the proximal section and the distal section are symmetrically arranged about the central axis.

5. The parallel motion mechanism according to any one of claims 1 to 4, wherein between the proximal end of the distal joint assembly and the distal end of the proximal joint assembly, the distal flexible member extends along a first straight line, and the proximal flexible member extends along a second straight line.

6. The parallel motion mechanism according to claim 5, wherein the first straight line and the second straight line are coplanar.

7. The parallel motion mechanism according to claim 5, wherein the first straight line is parallel to the second straight line, and is parallel to the central axis.

8. The parallel motion mechanism according to any one of claims 1 to 7, wherein the connector includes a first connection position where the distal flexible member is coupled and a second connection position where the proximal flexible member is coupled, and the first connection position and the second connection position are circumferentially spaced about the central axis.

9. The parallel motion mechanism according to claim 8, wherein, when the parallel motion mechanism is in a neutral state, the proximal section and the distal section extend parallel to the central axis, in a projection of the parallel movement mechanism along an extension direction of the central axis, the first connection position and the second connection position lie on a line connecting a position of the distal flexible member and a position of the proximal section.

10. The parallel motion mechanism according to claim 8, wherein, when the parallel motion mechanism is in a neutral state, the proximal section and the distal section extend parallel to the central axis, and when the parallel motion mechanism is projected onto a plane perpendicular to an extension direction of the central axis, the projection of the first connection position is coincident with the projection of the distal flexible member, and the projection of the second connection position is coincident with the projection of the proximal section.

11. The parallel motion mechanism according to claim 8, wherein the first connection position and the second connection position are spaced apart along an extension direction of the central axis.

12. The parallel motion mechanism according to any one of claims 1 to 11, further comprising:
a central tube extending along an extension direction of the central axis and is coupled between the proximal joint assembly and the distal joint assembly, a plurality of connectors are arranged circumferentially around the central tube.

13. The parallel motion mechanism according to claim 12, wherein the connector includes a guide portion coupling to the central tube and moveable relative to the central tube along the extension direction of the central axis.

14. The parallel motion mechanism according to claim 13, wherein the guide portion is configured as a guide ring surrounding an outer circumference of the central tube.

15. The parallel motion mechanism according to claim 14, wherein the connector further includes a connecting rod extending parallel to the central axis, and the guide ring is disposed at an end or a middle part of the connecting rod.

16. The parallel motion mechanism according to claim 15, wherein the connector further includes a first connection portion coupling to the distal flexible member and a second connection portion coupling to the proximal flexible member, the first connection portion radially protrudes from an outer peripheral surface of the guide ring or the connecting rod, and the second connection portion radially protrudes from the outer peripheral surface of the guide ring or the connecting rod.

17. The parallel motion mechanism according to any one of claims 1 to 16, wherein:
a plurality of connectors spaced apart from one another along an extension direction of the central axis; or
at least two connectors of the at least one connector are arranged in an overlapping manner along an extension direction of the central axis.

18. The parallel motion mechanism according to any one of claims 1 to 17, wherein the at least one plane comprises a first plane, the normal plane of the first plane is a first normal plane, and the plurality of drive assemblies comprise:
a first drive assembly and a second drive assembly, wherein the proximal section of the first drive assembly and the proximal section of the second drive assembly are disposed on two opposing sides of the first normal plane, respectively.

19. The parallel motion mechanism according to claim 18, wherein a distance between the proximal section of the first drive assembly and the first normal plane is equal to a distance between the proximal section of the second drive assembly and the first normal plane, and a distance between the distal section of the first drive assembly and the first normal plane is equal to a distance between the distal section of the second drive assembly and the first normal plane.

20. The parallel motion mechanism according to claim 18, wherein the proximal section of the first drive assembly and the proximal section of the second drive assembly are symmetrically arranged with respect to the central axis, and the distal section of the first drive assembly and the distal section of the second drive assembly are symmetrically arranged with respect to the central axis.

21. The parallel motion mechanism according to claim 18, wherein the at least one plane further comprises a second plane, wherein the second plane intersects the first plane at the central axis, the normal plane of the second plane is a second normal plane, and the plurality of drive assemblies further comprise:
a third drive assembly and a fourth drive assembly, wherein the proximal section of the third drive assembly and the proximal section of the fourth drive assembly are located on two opposing sides of the second normal plane, respectively.

22. The parallel motion mechanism according to claim 21, wherein a distance between the proximal section of the third drive assembly and the second normal plane is equal to a distance between the proximal section of the fourth drive assembly and the second normal plane, and a distance between the distal section of the third drive assembly and the second normal plane is equal to a distance between the distal section of the fourth drive assembly and the second normal plane.

23. The parallel motion mechanism according to claim 21, wherein the proximal section of the third drive assembly and the proximal section of the fourth drive assembly are symmetrically arranged with respect to the central axis, and the distal section of the third drive assembly and the distal section of the fourth drive assembly are symmetrically arranged with respect to the central axis.

24. A surgical instrument, comprising:
the parallel motion mechanism according to any one of claims 1 to 23;
an end effector coupled to the distal joint assembly of the parallel motion mechanism; and
a rear transmission device coupled to an end of the proximal flexible member.

25. The surgical instrument according to claim 24, wherein the end effector is configured as a hook, a scoop, a needle, forceps or scissors.

26. The surgical instrument according to claim 24 or claim 25, further comprising:
an end joint assembly, wherein the end effector is coupled to the distal joint assembly via the end joint assembly.

27. The surgical instrument according to claim 26, wherein the end joint assembly comprises a yaw joint base and a pitch joint base, the proximal end of the end effector is pivotably coupled to the distal end of the yaw joint base about a yaw axis, and the proximal end of the yaw joint base is pivotably coupled to the distal end of the pitch joint base about a pitch axis.

28. The surgical instrument according to claim 27, wherein the proximal end of the end effector is pivotably coupled to the distal end of the yaw joint base via a first clevis pin, and/or the proximal end of the yaw joint base is pivotably coupled to the distal end of the pitch joint base via a second clevis pin.

29. The surgical instrument according to claim 28, wherein the end effector comprises a first jaw and a second jaw, proximal ends of the first jaw and the second jaw are pivotably coupled to the yaw joint base via the first clevis pin, and the first jaw and the second jaw are independently pivotable about the first clevis pin relative to the yaw joint base.

30. The surgical instrument according to any one of claims 26 to 29, further comprising at least one pair of end drive cables, wherein ends of the at least one pair of end drive cables are coupled to the rear transmission device, and the at least one pair of end drive cables are configured to drive the end joint assembly and/or the end effector to move relative to the distal joint assembly.

31. The surgical instrument according to any one of claims 24 to 30, further comprising a cable electrically connected to the end effector, wherein the cable extends along the central axis when the parallel motion mechanism is in a neutral state.

32. A surgical robot, comprising:
a robotic arm, provided with an instrument drive device; and
the surgical instrument according to any one of claims 24 to 31, wherein the surgical instrument is removably coupled to the robotic arm, and the instrument drive device is operatively coupled to the rear transmission device to control the proximal flexible member via the rear transmission device.
